# EUROPEAN PATENT APPLICATION

(11) **EP 3 730 033 A1**
(43) Date of publication of application: **28.10.2020**
(21) Application number: 20170290.9
(22) Date of filing: 20.04.2020
(51) Int. Cl.: A61B 3/10

(54) **CORNEAL CONFOCAL MICROSCOPE**

(30) Priority: 23.04.2019 IT 201900006254
(71) Applicant: Nidek Technologies S.R.L., 35020 Albignasego (PD) (IT); Nidek Co., Ltd., Gamagori-Aichi, 443-0038 (JP)
(72) Inventor: PASCOLINI, Michele, I-35128 Padova (IT); MINOZZI, Mattia, I-35020 Legnaro (PD) (IT); CODOGNO, Nicola, I-35020 Albignasego (PD) (IT); PAJARO, Ronnye, I-30010 Campagna Lupia (VE) (IT); CARRARO, Federico, 35010 Villanova di Camposampiero (PD) (IT); TISO, Christian, I-35020 Albignasego (PD) (IT); TANASSI, Cesare, I-31053 Pieve di Soligo (TV) (IT)
(74) Representative: De Ros, Alberto

(57) **Abstract**

A corneal confocal microscope (1) is arranged to be used in a non-contact configuration with a cornea (C) of a human eye (E) and comprises a light source (5) configured to emit an illumination light beam (bl); a photodetector (10) configured to detect an imaging light beam (b2); and an optical assembly (20) configured to transmit the illumination light beam (bl) from the light source (5) to an observation volume (O) inside the cornea (C) and to transmit the imaging light beam (b2), backscattered from the observation volume (O), from the observation volume (O) to the photodetector (10). The optical assembly (20) is further configured to provide a depth of field comprised between 5 µm and 10 µm within the observation volume (O), and sized and/or configured so that the image (i2) of an imaging pinhole (40) in the observation volume (O) is larger than the image (il) of an illumination pinhole (30) in the observation volume (O). The microscope allows discriminating different layers inside the cornea and in each layer distinguishing singular cells in order to quantify and/or verify the "quality" (i.e. healthy or not-healthy) of the tissues and the cells. This information is useful for understanding, for example, if cornea laser treatment is safe or not and if cornea traditional surgery is safe or not. Furthermore, the microscope may provide eye banks with the possibility to analyse in detail if a cornea of a donor is good, healthy or not, and also to decide whether an explanted cornea should be held or rejected.

## Description

### FIELD of the INVENTION

The present invention relates to the field of confocal microscopy, which is a well-established way to investigate samples with very high spatial resolution.

In particular, the present invention relates to a corneal confocal microscope. Corneal confocal microscopy is a non-invasive clinical technique for the study, "in vivo" and "ex-vivo" of corneal cellular structure and it is currently used to investigate numerous corneal diseases.

More in detail, the principle of confocal microscopy relies on the use of pinholes placed in front of the source of light and of the light sensor in order to block out-of-focus light in image formation. This technique increases optical resolution and contrast compared to traditional microscopy.

Corneal microscopy is complicated by the structure of the eye and by the restrictions on the illumination source given by "in vivo" examination. Fluorescence light sources cannot be employed in illumination of the cornea and other sources have severe power limitations for safety reasons.

In addition to that, corneal microscopy is complicated by the fact that 10% of the light sent towards the eye is reflected at the air-cornea interface and generates noise. By contrast, due to the cornea own physical function of conveying light towards the retina, corneal layer back-scattering (containing the information) is in the order of 0.7% of the incoming light.

### BACKGROUND ART

In order to avoid or minimize the air-cornea reflection, known corneal confocal microscopy applications employ a liquid medium (oil, water or gel) placed between the objective and the eye.

This solution proves to be effective in reducing the noise enough and allowing effective imaging of the various corneal layers. However, the contact between the medium and the eye is a great discomfort to the patient. It may create pain, may require anaesthesia and raises a concern about the possibility of causing scratches on a patient's cornea.

There have been attempts at making non-contact corneal confocal microscopes. One of such attempts, by Tomey Corporation, utilizes a slit scanning system in order to collect images of the corneal tissue layers. In this design two independent slits are located in conjugated planes and are moved synchronously to produce the optical sectioning in real time.

The slit illumination has the advantage of decreasing the scan time compared to pinhole illumination. However, using slits in place of pinholes decreases the confocality of the microscope and allows the noise (in particular from the air-cornea reflection) to be detected by the light sensor, reducing the quality of the image. This is particularly disadvantageous when it comes to the resolution of the inner layers of the cornea.

Another solution that has been tested on the market for achieving non-contact corneal confocal microscopy consists in maximizing the confocality of both the light path and the image path.

This solution is effective in removing most of the noise from the reflection. However, tests have shown that maximizing the confocality of both paths leads to acquiring images of layers that are too thin for being effectively interpreted. According to patent document US4881808A, an ocular imaging system for use with a surgical laser device comprises an illumination assembly, an imaging assembly and a laser assembly all integrated together. These three assemblies use basically the same optical path, including a single same pinhole, and therefore they achieve the same level of confocality. In this case, this is an advantage as a surgeon can see exactly the effect of laser action.

According to patent document US2016278981A1, an ocular imaging system for use with a surgical laser device comprises an illumination assembly, an imaging assembly and a laser assembly. The system is designed to measure an optical break-through created in a tissue, beneath a tissue surface, by treating laser radiation. In practice, the system is designed to measure the size of plasma bubbles generated in the tissue by the laser radiation exactly where it is focused, and not to observe the surrounding tissue and its cells. Therefore, illumination, imaging and treatment relate exactly to the same small zone of the tissue,

### SUMMARY of the INVENTION

The aim of the subject-matter disclosed hereby to provide a corneal confocal microscope capable of solving the problems of known corneal confocal microscopes.

More in detail, the aim of the subject-matter disclosed hereby is to provide a non-contact corneal confocal microscope capable of acquiring detailed images of all layers of the cornea from the epithelium to the endothelium.

According to one aspect, the present invention relates to corneal confocal microscope arranged to be used in a non-contact configuration with a cornea of a human eye, according to annexed claim n° 1; in particular, the microscope comprises:
- a light source configured to emit an illumination light beam;
- a photodetector configured to detect an imaging light beam;
- an optical assembly configured to transmit the illumination light beam from the light source to an observation volume inside the cornea and to transmit the imaging light beam, backscattered from the observation volume, from the observation volume to the photodetector, wherein the optical assembly is further configured to provide a depth of field comprised between 5 µm and 10 µm within the observation volume.

Furthermore, the optical assembly is sized and/or configured so that the image of an imaging pinhole in the observation volume is larger than the image of an illumination pinhole in the observation volume, advantageously several times larger.

In this way, it is possible to obtain wider, well illuminated and in-focus images of a corneal layer investigated (and its cells) thanks to the diffusion of the light within the layer and thanks to the raster scan movement in the microscope. Advantageous features of the present invention are set out in annexed dependent claims that have to be considered an integral part of the present description.

The present invention allows discriminating different layers inside the cornea and in each layer distinguishing singular cells in order to quantify and/or verify the "quality" (i.e. healthy or not-healthy) of the tissues and the cells. This information is useful for understanding, for example, if cornea laser treatment is safe or not and if cornea traditional surgery is safe or not.

Furthermore, the present invention may provide eye banks with the possibility to analyse in detail if a cornea of a donor is good, healthy or not, and also to decide whether an explanted cornea should be held or rejected.

### LIST of DRAWINGS

The present invention will become more apparent from the following description to be considered in conjunction of with the annexed figure wherein:
Fig. 1 shows a section view of an eye and a detailed section view of a cornea;
Fig. 2A-2E show microscopic images of different layers of a cornea;
Fig. 3 shows a schematic view of a corneal confocal microscope according to the present invention;
Fig. 4 shows a schematic planar view of images formed in a cornea of an image pinhole and an illumination pinhole of a corneal confocal microscope according to the present invention;
Fig. 5 shows a schematic section view of a cornea examined with a corneal confocal microscope according to the present invention.

### DETAILED DESCRIPTION of the INVENTION

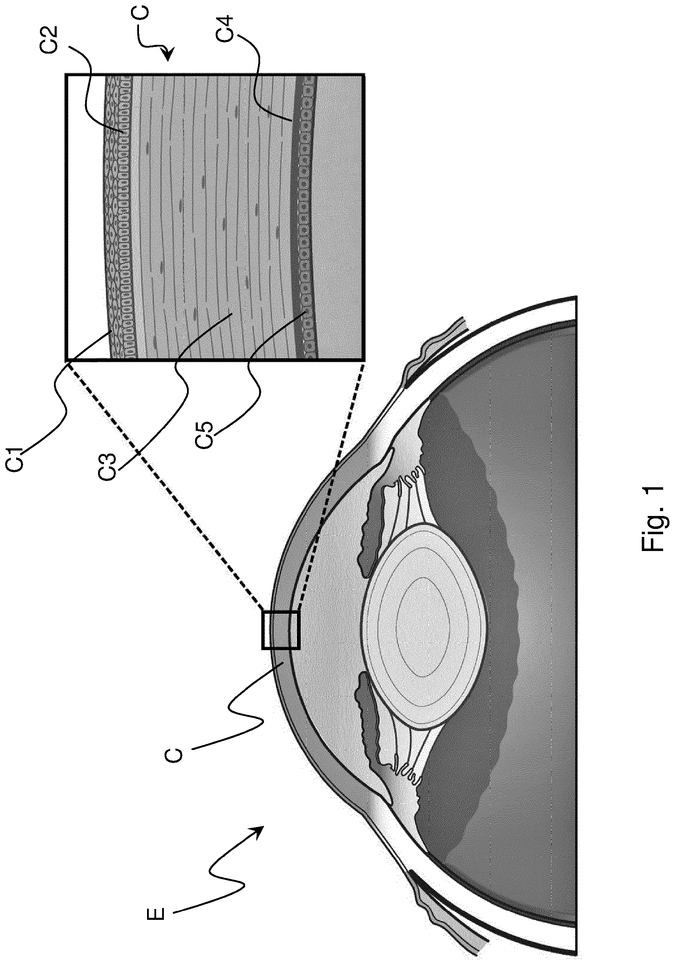

Fig. 1 shows a section view of an eye "E" and its cornea "C". The cornea is the almost perfectly transparent front part of the eye that covers the iris, pupil, and anterior chamber. Together with the anterior chamber and lens, the cornea refracts light and accounts for approximately two-thirds of the eye's total optical power.

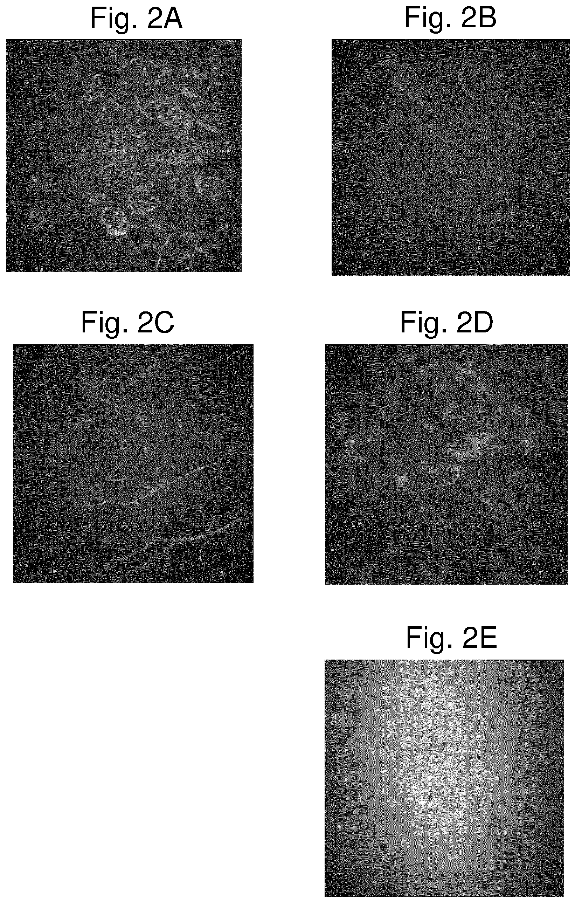

Fig. 1 also shows a detail of the layers which form the cornea "C": the epithelium "C1", Bowman's layer "C2", the stroma "C3", Descemet's membrane "C4", and the endothelium "C5". Fig. 2 shows planar views of the same layers acquired by a confocal microscope.

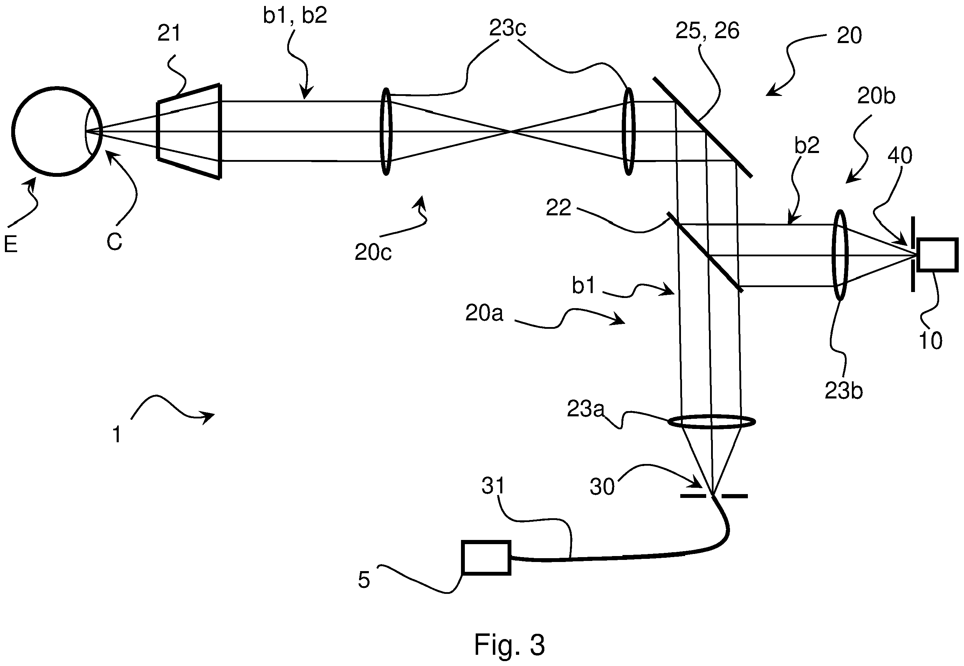

Fig. 3 shows a schematic view of the optical layout of a corneal confocal microscope according to the present invention, indicated with the numerical reference 1 and referred to as "microscope 1" in the following parts of this description.

The microscope 1 is arranged to be used in a non-contact configuration with a cornea "C" of an eye "E", which is also schematically shown in Fig. 3.

In the microscope 1 comprises a light source 5 configured to emit an illumination light beam "b1", a photodetector 10 configured to detect an imaging light beam "b2" and an optical group 20 coupled with the light source 5 and the photodetector 10 to transmit the illumination light beam "b1" and the imaging light beam "b2". Preferably, the light source 5 consists in a red light laser or in a super-luminescent diode. In the first case, the red light laser is configured to emit light at a wavelength comprised between 620 nm and 690 nm. In the second case, the super-luminescent diode (SLD) is configured light and/or infrared radiation at a wavelength (or at a spectrum of wavelengths) comprised between 620 nm and 770 nm, preferably between 680 nm and 770 nm.

Advantageously, light and infrared radiation between 620 nm and 770 nm benefit from an increased transmittance of the cornea compared with radiations at lower wavelengths. The transmittance of the cornea is further increased in the interval between 680nm and 770 nm, therefore a super-luminescent diode generating light at these wavelengths is the preferred solution. Radiation sources at wavelengths higher than 770 nm are not taken into consideration for preventing damages to the cornea itself.

Preferably, the photodetector 10 comprises an avalanche photodiode (APD) configured to detect signals in the range of picoWatt, positioned on the path of the imaging light beam "b2" in order to detect it and convert it into an electrical signal, and a transimpedance amplifier connected with the APD in order to amplify its signal.

As shown in Fig. 3, the optical assembly 20 comprises a plurality of lenses and other optical devices. Such lenses and optical devices are configured to transmit the illumination light beam "b1" from the light source 5 to an observation volume "O" inside the cornea "C" and transmit the imaging light beam "b2" from the observation volume "O" to the photodetector 10.

The observation volume "O" can be positioned in any layer of the cornea "C" from the epithelium "C1" to the endothelium "C5" or at an interface between layers.

Advantageously, the optical assembly 20 is configured to provide a depth of field inside the observation volume "O" comprised between 5 µm and 10 µm. This depth of field has been found to be enough for achieving good image characteristics in terms of resolution and contrast in non-contact configuration, while at the same time being deep enough for capturing the structures of interest inside the cornea "C", at any layer of the cornea "C" itself (including the endothelium "C5"). Such depth of field is achieved through the characteristics and configuration described below.

The illumination light beam "b1" directed by the optical assembly 20 enters in the cornea "C" through the air-cornea interface and converges in the observation volume "O". A fraction of the radiation forming the illumination light beam "b1" is backscattered from the observation volume "O" towards the microscope 1 through the air-cornea interface. This backscattered radiation forms the imaging light beam "b2", which is some orders of magnitude smaller than the illumination light beam "b1".

Preferably, the light source 5 is set in such a way that a power equal or less than 2.5 mW enters the cornea, accounting for all the losses through the optical group 20, in order to prevent damages to the eye.

The optical assembly 20 comprises an objective 21 arranged to face the cornea "C" at a predetermined distance from the cornea "C" itself and configured to focus the illumination light beam "b1" in the observation volume "O" and to collect the imaging light beam "b2" from the observation volume "O". The position of the objective 21 with respect to the eye "E" and its configuration determines the axial position of the observation volume "O" inside the cornea (that may be adjusted according to the desired cornea layer to be imaged). The objective 21 may be removably connected to the structure of the microscope 1 and may be replaceable. The objective 21 is the part of the optical assembly 20 which is positioned closest to the eye "E" during the examination procedure. Preferably, the objective 21 is configured so that the distance between the objective 21 and the cornea "C" during the examination comprised between 5 mm and 20 mm, preferably between 10 mm and 14 mm, to stay well apart from the patient cornea and eyelids. In other words, the optical characteristics of the objective are selected in order to position the observation volume "O" at a distance comprised between 5 mm and 20 mm, preferably between 10 mm and 14 mm form the objective 21 itself.

In the preferred embodiment, the focal length of the objective 21 is about 9 mm and its Numerical Aperture is about 0.4 and its working distance is 12 mm. Also, the objective can move in front of the eye by means of a motor with micrometric steps to provide a z-scan of the cornea.

Additionally, the optical assembly 20 comprises a beam splitter 22 and has an illumination arm 20a transmitting the illumination light beam "b1" from the light source 5 to the beam splitter 22, an imaging arm 20b transmitting the imaging light beam "b2" from the beam splitter 22 to the photodetector 10 and a combined arm 20c transmitting the illumination and imaging light beams "b1" and "b2" between the beam splitter 22 and the objective 21 superimposed on the same path. The optical assembly 20 has an illumination pinhole 30 located in the illumination arm 20a on the path to the illumination light beam "b1", so that the illumination light beam "b1" passes through it in order to achieve confocality. Preferably, the illumination pinhole 30 has a width comprised between 2 µm and 3 µm, more preferably about 2.5 µm.

The illumination pinhole 30 may be a conventional hole across a body or may be formed by the core of an optical fiber. In the embodiment of Fig. 3, the optical assembly 20 has an illumination optical fiber core 31 (single mode) having a first end coupled with the light source 5 and a second end facing the illumination arm 20a, defining the illumination pinhole 30. Advantageously, the fiber core is better than a conventional pinhole because it allows to obtain a more uniform beam. The optical assembly 20 comprises a plurality of lenses 23a and 23c on the path of the illumination light beam "b1" between the illumination pinhole 30 and the observation volume "O".

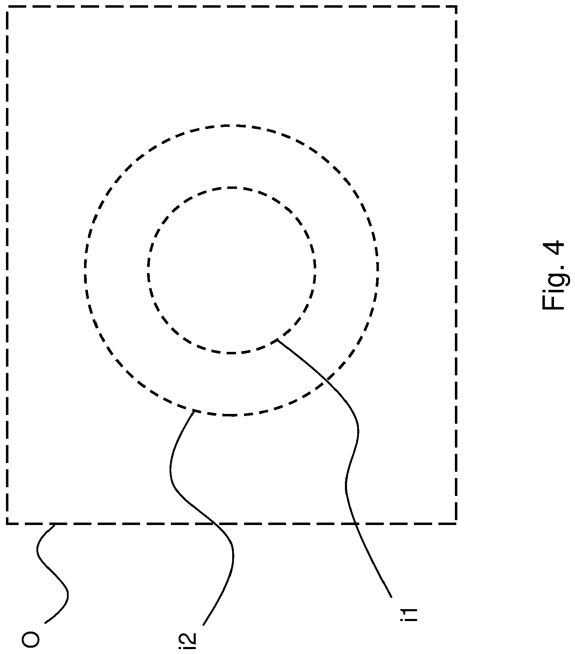

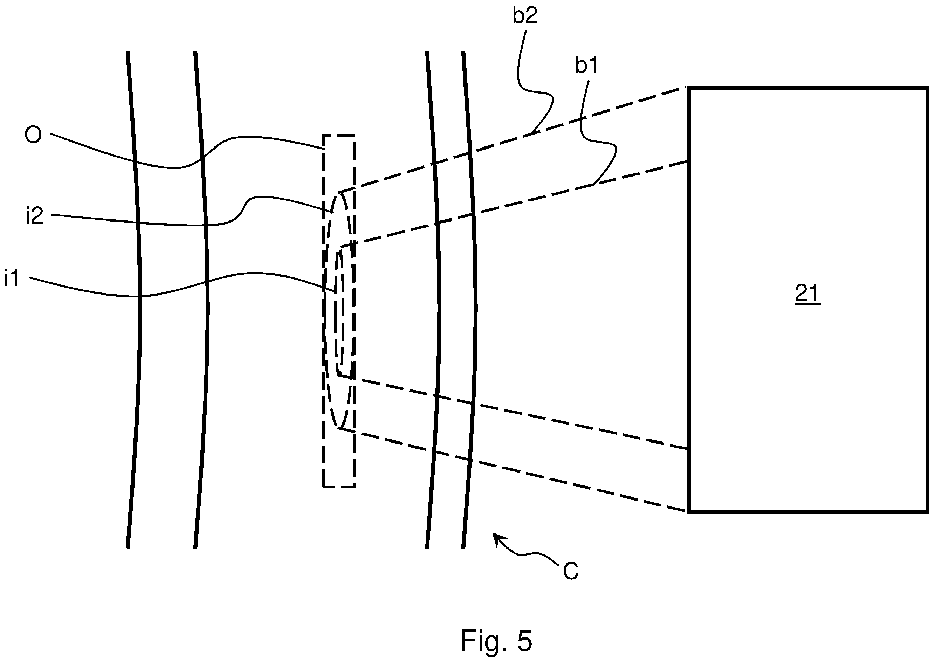

The illumination light beam "b1" projects an image "i1" of the illumination pinhole 30 into the observation volume "O", which is shown in Fig. 4 and Fig. 5 (these figures show a situation corresponding to a fictitious stop of the raster scan). The size of the image "i1" is determined by the size of the illumination pinhole 30 itself and by the magnifications (intended as both magnifications and minifications for the purpose of this description) applied by lenses 23a and 23c and by the objective 21 on the illumination light beam "b1". According to typical embodiments, image "i1" has a width comprised between 0.6 µm and 1.0 µm, preferably about 0.8 µm.

Preferably, the optical assembly 20 is configured in order to achieve a diffraction-limited image "i1" of the illumination pinhole 30 into the observation volume "O", which is its smallest possible size. In other words, the combined lenses and other optical devices in the path of the illumination light beam "b1", including the objective 21, are configured to reduce the section of the illumination light beam "b1" to the size of the Airy disk when the illumination light beam "b1" enters the observation volume "O".

In particular, the optical devices affecting the illumination light beam "b1" include at least a lens 23a placed in the illumination arm 20a, at least two lenses 23c placed in the combined arm 20c and the objective 21. Lenses 23a and 23c may be replaced by any number of lenses arranged in the illumination arm 20a in order to achieve the desired overall magnification factor.

The optical assembly 20 has an imaging pinhole 40 located in the imaging arm 20b on the path of the imaging light beam "b2", so that the imaging light beam "b2" passes through it in order to achieve confocality. Preferably, the imaging pinhole 40 has a width comprised between 20 µm and 30 µm, more preferably about 25 µm. According to typical embodiments, the imaging pinhole 40 has a width comprised between 23 µm and 27 µm. Depending on magnification of the optical assembly (intended as both magnification and minification), the width of the imaging pinhole may vary quite a lot: an absolute minimum may be 5 µm and an absolute maximum may be 30 µm.

As already explained, the optical assembly 20 is sized and/or configured so that the imaging pinhole 40 has a width larger than that of the illumination pinhole 30, and that the image i2 of the imaging pinhole 40 is larger than the image i1 of the illumination pinhole 30; furthermore, the image i1 of the illumination pinhole 30 in the observation volume O is diffraction-limited.

In particular and advantageously, the optical assembly 20 is sized and/or configured so that the image i2 of the imaging pinhole 40 is M times larger than the image i1 of the illumination pinhole 30, M being in the range between 2 and 6, preferably between 3 and 5, more preferably being approximately equal to 4.

In particular and advantageously, the imaging pinhole 40 is N times larger than the size based on diffraction limit (that may be for example about 6.5 µm), N being in the range between 2 and 6, preferably between 3 and 5, more preferably being approximately equal to 4.

Like the illumination pinhole 30, the imaging pinhole 40 may be a conventional hole across a body or may be formed by an optical fiber core. In the embodiment of Fig. 3, the optical assembly 20 comprises a body defining the imaging pinhole 40 in front of the photodetector 10.

The optical assembly 20 comprises a plurality of lenses 23b and 23c on the path of the imaging light beam "b2" between the observation volume "O" and the imaging pinhole 40. Lenses 23c, positioned in the combined arm 20c, are shared with the illumination light beam "b1". Lenses 23b, positioned in the imaging arm 20b, are passed through by the imaging light beam "b2" alone.

The imaging light beam "b2" projects a virtual image "i2" of the imaging pinhole 40 into the observation volume "O", which is shown in in Fig. 4 and Fig. 5 (these figures show a situation corresponding to a fictitious stop of the raster scan). The size of the image "i2" is determined by the size of the imaging pinhole 40 itself and by the magnifications (intended as both magnifications and minifications for the purpose of this description) applied by the lenses and other optical devices on the imaging light beam "b2" (as well as by the diffraction limit). According to typical embodiments, image "i2" has a width comprised between 2.4 µm and 4.0 µm, preferably about 3.2 µm.

Preferably, the optical assembly 20 and the imaging pinhole 40 are configured so that the image "i2" of the imaging pinhole 40 into the observation volume "O" is wider than the image "i1" of the illumination pinhole 30. In other words, the imaging light beam "b2" is less confocal than the illumination light beam "b1" and its section inside the observation volume "O" is greater than the Airy disk.

In particular, the optical devices affecting the imaging light beam "b2" include at least a lens 23b placed in the imaging arm 20b, and the lenses 23c placed in the combined arm 20c and the objective 21. Lenses 23b and 23c may be replaced by any number of lenses arranged in the imaging arm 20b in order to achieve the desired overall magnification factor.

Advantageously, the size of the image "i1" of the illumination pinhole 30 into the observation volume "O" determines the almost perfect confocality of the illumination light beam "b1" and allows good acquired image characteristics in terms of resolution and contrast. On the other hand, the size of the image "i2" of the imaging pinhole 40 into the observation volume "O" determines a slightly less than perfect confocality of the imaging light beam "b2" and allows a deeper depth of field than what would be achievable with a higher confocality of the imaging light beam "b2", while not dropping the resolution and contrast of the acquired image significantly. In particular, the described configuration avoids most of the reflection at the air-cornea interface.

It is to be noted that the sized set out in the preceding paragraphs may vary depending on magnifications of the optical assembly (intended as both magnification and minification).

Preferably, the optical assembly 20 comprises a resonant mirror 25 and a galvanometer mirror 26 coupled with the photodetector 10 in order to scan observation volume "O" in a raster way. Both the resonant and galvanometer mirrors 25 and 26 are positioned in the combined arm 20c of the optical group. Preferably, the distance between a resonant mirror 25 and a galvanometer mirror 26 is equal or smaller than 6 mm. A short distance between the between the resonant and galvanometer mirrors 25 and 26 is important because it makes unnecessary to refocus the illumination and imaging light beams "b1" and "b2" between them.

Preferably, lenses (or group of lenses) 23a, 23b and 23c are dimensioned according to the following additional criteria, which may complement the features described above. The lenses 23c are required to adjust the dimension of the illumination and imaging light beams "b1" and "b2" from the size of mirrors 25 and 26 to the objective 21 aperture. The magnification of lens(es) 23a is configured to adjusts the diameter of the illumination light beam "b1" so that it matches the aperture of the objective 21 for the best illumination condition of the sample. In the preferred embodiment, the overall focal length of lens(es) 23a is about 15.8 mm, the overall focal length of lens(es) 23b is about 40 mm and, the focal length of lenses 23c is 75 mm and 40 mm respectively.

Preferably, each image acquired by the photodetector 10 has a resolution higher than 500'000 pixels and a field of view comprised between 200 µm and 800 µm. In particular, each image has a resolution of about 1 Megapixels and a field of view of about 400 µm. Field of view and resolution are chosen so to image a whole cornea layer (or a substantial part of a cornea layer) and observe cells having a minimum size of few µm.

Preferably, the speed of the resonant mirror 25, the speed of the galvanometer mirror 26 and the bandwidth of the APD are configured for allowing a frame rate higher than 5 images per seconds, preferably higher than 10 images per seconds. In particular, the frame rate is about 18 images per second. In order to achieve this, in the preferred embodiment the resonant mirror 25 has a frequency of about 12 kHz.

Advantageously, such resolution and speed allow continuous acquisition of good quality images which minimizes the issues related to inevitable movements of the patient.

Preferably, the optical assembly 20 is rigidly mounted on a support which sets the distance and the relative position between the components, with the exception of the objective 31 which may be movable by its motor parallel to the imaging and illumination light beams "b1" and "b2". Additionally, the microscope 1comprises a sagittal robot configured for moving the support in a spherical plane around the observation volume "O". Advantageously, the sagittal robot permits to move the acquisition everywhere around the cornea "C" and to repeat the examination in the same position. This feature allows the follow up of the patient examination.

## Claims

1. Corneal confocal microscope (1) arranged to be used in a non-contact configuration with a cornea (C) of a human eye (E), said microscope (1) comprising:
- a light source (5) configured to emit an illumination light beam (b1);
- a photodetector (10) configured to detect an imaging light beam (b2); and
- an optical assembly (20) configured to transmit the illumination light beam (b1) from the light source (5) to an observation volume (O) inside said cornea (C) and to transmit the imaging light beam (b2) from the observation volume (O) to the photodetector (10), said imaging light beam (b2) being backscattered from said observation volume (O);
wherein said optical assembly (20) is further configured to provide a depth of field comprised between 5 µm and 10 µm within said observation volume (O); wherein said optical assembly (20) has an illumination pinhole (30) positioned on a path of the illumination light beam (b1) and an imaging pinhole (40) positioned on a path of the imaging light beam (b2),
wherein said optical assembly (20) is sized and/or configured so that the image (i1) of the illumination pinhole (30) in the observation volume (O) is diffraction-limited;
wherein said imaging pinhole (40) has a width larger than a width of said illumination pinhole (30);
wherein said optical assembly (20) being sized and/or configured so that the image (i2) of said imaging pinhole (40) is larger than the image (i1) of said illumination pinhole (30).

2. Microscope (1) according to claim 1, wherein said optical assembly (20) is sized and/or configured so that the image (i2) of said imaging pinhole (40) is M times larger than the image (i1) of said illumination pinhole (30), M being in the range between 3 and 5, preferably being approximately equal to 4.

3. Microscope (1) according to claim 1 or 2, wherein said imaging pinhole (40) is N times larger than size based on the diffraction limit, N being in the range between 3 and 5, preferably being approximately equal to 4.

4. Microscope (1) according to one or more of the claims from 1 to 3, wherein said image (i1) of said illumination pinhole (30) has a width comprised between 0.6 µm and 1.0 µm, preferably about 0.8 µm.

5. Microscope (1) according to one or more of the claims from 1 to 4, wherein said image (i2) of said imaging pinhole (40) has a width comprised between 2.4 µm and 4.0 µm, preferably about 3.2 µm.

6. Microscope (1) according to one or more of the claims from 1 to 5, wherein said illumination pinhole (30) has a width comprised between 2 µm and 3 µm, preferably about 2.5 µm.

7. Microscope (1) according to one or more of the claims from 1 to 6, wherein said imaging pinhole (40) has a width comprised between 20 µm and 30 µm, preferably about 25 µm.

8. Microscope (1) according to one or more of claims from 1 to 7, wherein said optical assembly (20) comprises an illumination optical fiber core (31) coupled with the light source (5) in order to define said illumination pinhole (30) and/or said optical assembly (20) comprises an imaging optical fiber core coupled with the photodetector (10) in order to define said imaging pinhole (40).

9. Microscope (1) according to one or more of the preceding claims, wherein said optical assembly (20) has an end configured to face said cornea (C), said end of the optical assembly (20) being configured to emit said illumination light beam (b1) towards the observation volume (O) and to collect the imaging light beam (b2) from the observation volume (O); the distance between said end of the optical assembly (20) and the observation volume (O) being comprised between 5 mm and 20 mm, preferably between 10 mm and 14 mm.

10. Microscope (1) according to one or more of the preceding claims, wherein said light source (5) is configured to emit light at a wavelength comprised between 620 nm and 770 nm, preferably between 680nm and 770 nm.

11. Microscope (1) according to claim 10 wherein said light source (5) is a red light laser or a super-luminescent diode.

12. Microscope (1) according to one or more of the preceding claims, wherein said optical assembly (20) comprises a resonant mirror (25) and a galvanometer mirror (26) configured to scan said observation volume (O) in a raster configuration.

13. Microscope (1) according claim 12, wherein said photodetector (10) comprises an avalanche photodiode and wherein the speed of the resonant mirror (25), the speed of the galvanometer mirror (26) and the bandwidth of the avalanche photodiode are configured for allowing a frame rate higher than 5 images per seconds, preferably higher than 10 images per seconds, each image having a resolution higher than 500'000 pixels.

14. Microscope (1) according to one or more of the previous claims, further comprising:
- a support rigidly coupled with the optical assembly (20);
- a sagittal robot configured for moving said support in a spherical plane around said observation volume (O).
